# EUROPEAN PATENT APPLICATION

(11) **EP 2 423 321 A2**
(43) Date of publication of application: **29.02.2012**
(21) Application number: 11168962.6
(22) Date of filing: 04.01.2007
(51) Int. Cl.: C12Q 1/25, G01N 33/50, G01N 33/574, C07K 16/30

(54) **Use of HE4 and other biochemical markers for assessment of endometrial and uterine cancers**

(30) Priority: 04.01.2006 US 756131 P
(62) Divisional of application: 07717842.4
(71) Applicant: Fujirebio America, Inc., Wilmington, DE 19808 (US)
(72) Inventor: Moore, Richard, Cranston, RI 02921 (US); Somers, Elizabeth, Unionville, PA 19375 (US); Allard, Jeffrey W., Harleysville, PA 19438 (US)
(74) Representative: Walker, Ross Thomson

(57) **Abstract**

The disclosure relates to use of the HE4/HE4a marker(s) together with one or more other markers to assess ovarian cancers in a patient.

## Description

### BACKGROUND OF THE DISCLOSURE

The disclosure relates generally to the field of diagnosis, grading, staging, and prognosis of cancer. More particularly, this disclosure relates to the field of endometrial and uterine cancers. This disclosure further relates to the field of diagnosis, grading, staging, and prognosis using protein expression.

Cancer includes a broad range of diseases, affecting approximately one in four individuals worldwide. The severity of the adverse impact of cancer is profound, influencing medical policy and procedure as well as society generally. Because a hallmark of many types of cancer is rapid and unregulated proliferation of malignant cells, an overarching problem in improving approaches to cancer is the need for early detection and diagnosis. Early detection is well regarded as the best means to reduce cancer mortality. In response, numerous attempts have been made to develop accurate and reliable criteria for diagnosing the presence of a malignant condition. In particular, investigations have been directed to the use of serologically defined antigenic markers known as tumor associated antigens, which are either uniquely expressed by cancer cells or are present at markedly higher levels in subjects having a malignant condition.

However, due to the high heterogeneity of tumor associated antigen expression, for example the extreme diversity of carcinoma antigens, there is a need for additional tumor markers that are useful in cancer diagnosis. Many monoclonal antibodies reactive with carcinoma associated antigens are known. Such monoclonal antibodies bind to a variety of different carcinoma-associated antigens including glycoproteins, glycolipids, and mucins. Many such monoclonal antibodies recognize tumor-associated antigens that exhibit restricted, expression on some, but not other, tumors originating in a given cell lineage or tissue type.

There are relatively few examples of tumor associated antigens that appear to be useful for identifying a particular type of malignancy. Monoclonal antibody 872.3, for example, specifically binds to a high molecular mass (>106 Da) tumor-associated mucin antigen that is selectively expressed on a number of different carcinomas, including most if not all ovarian carcinomas and an overwhelming majority of non-small cell lung carcinomas, colon carcinomas and breast carcinomas. Nevertheless, detection of cell-associated tumor markers such as the mucin antigen recognized by B72.3 following surgical resection of a tumor may be of limited usefulness for diagnostic screening, in which early detection of a malignant condition prior to accumulation of substantial tumor mass is preferred.

An alternative to the diagnosis of a particular type of cancer by screening surgically resected specimens for tumor associated antigens, where invasive surgery is usually indicated only after detection of an accumulated tumor mass, would be to provide compositions and methods for detecting such antigens in samples obtained from subjects by non-invasive or minimally invasive procedures. In ovarian, endometrial, and other carcinomas, for example, there are currently a number of soluble tumor associated antigens that are detectable in samples of readily obtained biological fluids such as serum or mucosal secretions. One such marker is CA125, a carcinoma-associated antigen that is also shed into the bloodstream, where it is detectable in serum (e.g., Bast et al., 1983 N. Eng. J. Med. 309:883; Lloyd et al., 1997 Int. J. Canc. 71:842). CA125 levels in serum and other biological fluids have been measured along with levels of other markers, for example, carcinoembryonic antigen (CEA), squamous cell carcinoma antigen (SCC), tissue polypeptide specific antigen (TPS), sialyl TN mucin (STN) and placental alkaline phosphatase (PLAP), in an effort to provide diagnostic and/or prognostic profiles of ovarian, endometrial, and other carcinomas (e.g., Sarandakou et al., 1997 Acta Oncol. 36:755; Sarandakou et al., 1998 Eur. J. Gynaecol. Oncol. 19:73; Meier et al., 1997 Anticanc. Res. 17(4B):2945; Kudoh et al., 1999 Gynecol. Obstet. Invest. 47:52; Ind et al., 1997 Br. J. Obstet. Gynaecol. 104:1024; Bell et al. 1998 Br. J. Obstet. Gynaecol. 105:1136; Cioffi et al., 1997 tumor 83:594; Meier et al. 1997 Anticanc. Res. 17(4B):2949; Meier et al., 1997 Anticanc. Res. 17(4B):3019).

Elevated levels of serum CA125 alone or in combination with other known indicators, however, do not provide a definitive diagnosis of malignancy, or of a particular malignancy such as ovarian or endometrial carcinoma. For example, elevated CA125, CEA and SCC in vaginal fluid and serum correlate most strongly with inflammation in benign gynecological diseases, relative to cervical cancer and genital tract cancers (e.g., Moore et al., 1998 Infect. Dis. Obstet. Gynecol. 6:182; Sarandakou et al., 1997 Acta Oncol. 36:755). Elevated serum CA125 can also accompany neuroblastoma, and elevated CEA and SCC levels can accompany colorectal cancer. Another marker, the differentiation antigen mesothelin, is expressed on the surfaces of normal mesothelial cells and also on certain cancer cells, including epithelial ovarian tumors and mesotheliomas. Compositions and methods pertaining to mesothelin (Chang et al., 1992 Canc. Res. 52:181; Chang et al., 1992 Int. J. Canc. 50:373; Chang et al., 1992 Int. J. Canc. 51:548; Chang et al., 1996 Proc. Nat. Acad. Sci. USA 93:136; Chowdhury et al., 1998 Proc. Nat. Acad. Sci. USA 95:669; Yamaguchi et al., 1994 J. Biol. Chem. 269:805; Kojima et al., 1995 J. Biol. Chem. 270:21984) and structurally related mesothelin related antigen (MRA; see, e.g., Scholler et al., 1999 Proc. Nat. Acad. Sci. USA 96:11531) are known in the art, including uses in cancer detection and therapies as described in WO 00/50900 and in U.S. application Ser. No. 09/513,597. There is a compelling need for additional markers useful in multiple marker diagnostic screening.

### SUMMARY OF THE DISCLOSURE

The subject matter of this disclosure includes a method of assessing whether a patient is afflicted with an endometrial or uterine cancer. The method comprises assessing expression of HE4 in a sample (e.g., a solid tissue sample, a body fluid sample, or a sample of a fluid that has been contacted with a relevant portion of the patient's body) obtained from the patient. Elevated expression of HE4 is an indication that the patient is afflicted with an endometrial or uterine cancer. If desired, the level of HE4 expression can be compared with a reference value, such as a reference value that corresponds to HE4 expression in patients who are not afflicted with an endometrial or uterine cancer. Alternatively, expression of HE4 in the sample can be compared with expression of HE4 in an earlier sample obtained at an earlier time from the patient.

In a preferred embodiment, the method further comprises assessing expression of a second marker (e.g., CA125 and/or SMRP) in the sample. Elevated expression of the second marker is a further indication that the patient is afflicted with an endometrial or uterine cancer.

The disclosure also relates to a method of assessing the response of a patient afflicted with an endometrial or uterine cancer to a treatment. The method comprises assessing expression of HE4 in samples obtained from the patient at different times during treatment. Decreased expression of HE4 at the later time (or a decrease in expression over time) indicates that the patient is responding to the treatment. Expression of a second marker (e.g., CA125 and/or SMRP) can also be assessed in the sample. Decreased expression of the second marker is a further indication that the patient is responding to the treatment.

The disclosure further relates to a method of assessing recurrence in a patient who has been treated for an endometrial or uterine cancer. The method comprising assessing expression of HE4, alone or in combination with one or more second markers, in samples obtained from the patient following treatment. Elevated expression of HE4 indicates that the endometrial or uterine cancer is recurring in the patient, and elevated expression of the second marker is a further indication that the endometrial or uterine cancer is recurring in the patient. Expression of HE4 can, for example, be assessed multiple times following the treatment. Increasing expression of HE4 over time indicates that the endometrial or uterine cancer is recurring in the patient.

In another embodiment, the disclosure relates to a method of assessing the likelihood that a patient will develop an endometrial or uterine cancer. The method comprising assessing expression of HE4, alone or in combination with one or more second markers, in a sample obtained from the patient. Elevated expression of HE4 is correlated with increased likelihood that the patient will develop an endometrial or uterine cancer, and elevated expression of the second marker is a further indication that the patient will develop an endometrial or uterine cancer.

In yet another embodiment, the disclosure pertains to a method of staging and/or grading a tumor in a patient afflicted with an endometrial or uterine cancer. The method comprising assessing expression of HE4, alone or in combination with one or more second markers, in a sample obtained from the patient. Increasing expression of HE4 (and the second markers, if assessed) indicates a more advanced stage of the cancer and/or a higher grade of the of the cancer.

Still another aspect of the subject matter disclosed herein pertains to a method for assessing the need of a patient diagnosed with an endometrial or uterine cancer for a therapeutic intervention (e.g., chemotherapy). The method comprising assessing expression of HE4, alone or in combination with one or more second markers, in a sample obtained from the patient. Elevated expression of HE4 (and the second markers, if assessed) indicates need of the patient for the therapeutic intervention.

### BRIEF SUMMARY OF THE SEVERAL TABLES

Table 1 is a summary of area under curve (AUC) analysis of ROC (Receiver Operating Characteristic) curve data obtained in the experiments described herein in Example 1.

Table 2 is the mean values of serum tumor markers and the staging determined for each set of values grouped by each marker analyzed as described in Example 2.

Table 3 is the ROC-AUC calculated for each tumor marker as described in Example 2

Table 4 is the logistic regression analysis at a specificity of 90% for each tumor marker independently in the combinations set forth in Example 2

Table 5 is the logistic regression analysis at a specificity of 95% for each tumor marker independently in the combinations set forth in Example 2

Table 6 is the logistic regression analysis at a specificity of 98% for each tumor marker independently in the combinations set forth in Example 2

### DETAILED DESCRIPTION

The disclosure relates to the discovery that the HE4a antigen is an indicator of the existence, grade, and stage of endometrial and uterine ("corpus uterine") cancers.

The subject matter of this disclosure relates generally to assessment of endometrial and uterine cancer in women using the HE4 marker or a combination of HE4 and other biological markers. Assessing HE4 with one or more additional makers of endometrial and uterine cancers (especially markers CA125 and SMRP) can be used to diagnose occurrence of such cancers in a human patient, and to assess the response of such cancers to treatments of various types. Furthermore, assessment of these markers can be used to monitor recurrence of such cancers in a patient or to assess the likelihood that a patient will develop such cancers.

Approximately 40,880 new cases of uterine cancer are diagnosed in the US annually resulting in 7,310 deaths. Uterine cancer is surgically staged with 75% of patients presenting with stage I disease (Kottmeier, H.L., Int. J. Gynaecol. Obstet. 1971; 9:172). Patients diagnosed with stage I disease that have intermediate to high risk factors have a recurrence rate of 20 to 30% (Keys et al., 2004, Gynecol. Oncol.; 92(3):744-751; Creutzberg et al., 2003, Gynecol. Oncol., 89(2): 201-209). The serum CA125 levels are elevated in about 75% of patients with advanced stage III and IV disease and is elevated in only 17% of patients with early stage I and II disease (Niloff et al, 1984, Am. J. Obstet. Gynecol., 148(8): 1057-1058; Berchuck et al., 1989, Cancer Res., 49(8):2091-2095; Olt et al., 1990, Obstet. Gynecol. Surv., 45: 570-577). Elevated serum CA125 levels have been correlated with endometrial cancer stage and spread of the tumor to the cervix, lymph nodes and peritoneal surfaces (Ginath et al., 2002, Int. J. Gynecol. Cancer, 12(4): 372-375; Vuento et al., 1997, Gynecol. Oncol., 64(1): 141-146; Sood et al., 1997, Obstet. Gynecol. 90(3): 441-447; Hsieh et al., 2002, Gynecol. Oncol., 86(1): 28-33; Powell et al., 2005, J. Reprod. Med., 50(8): 585-590). CA125 currently is the most useful serum tumor marker for the detection of recurrent disease. However, the use of CA125 for the detection of recurrent disease is limited at best. Only 10 to 20% of patients with early stage disease and 25% of patients with asymptomatic recurrent disease have an elevated CA125 (Niloff et al., 2002; Duk et al., 1986, Am. J. Obstet. Gynecol., 155(5): 1097-1102). There have been a number of novel serum tumor markers studied for their clinical utility in endometrial cancer including CA15.3, CA19.9, CA72.4, CEA, OVX1 and M-CSF (Cherchi et al. 1999, Eur. J. Gynaecol. Oncol., 20(4): 315-317; Takeshima et al, 1994, Gynecol Oncol 1994; 54(3):321-326; Hareyama et al., 1996, J. Clin. Pathol., 49(12): 967-970; Olt et al., 1996, Am. J. Obstet. Gynecol., 174(4):1316- 1319; Beck et al., 1997, Gynecol. Oncol., 65(2):291-296) More recently proteomic analysis have been employed to identify novel makers for endometrial cancer such as chaperonin 10 (Yang et al., 2004, J. Proteome Res., 3(3):636- 643). Very few studies have employed the strategy of multiple marker analysis for the use in endometrial cancers. Prior to disclosure of the methods described herein, the most promising combination to date is CA125 with CA19.9 for the detection of recurrent disease (Lo et al., 1999, Cancer Detect. Prev. 23 (5):397- 400).

Definitions

As used herein by the term a "sample" is meant material which can be specifically related to a patient and from which specific information about the patient can be determined, calculated or inferred. A sample can be composed in whole or in part of biological material from of the patient. A sample can also be material that has contacted the patient in a way that allows tests to be conducted on the sample which provides information about the patient. A sample may also be material that has contacted other material that is not of the patient but allows the first material to then be tested to determine information about the patient, e.g. a sample can be a wash of a probe or scalpel. A sample can contact sources of biologic material other than the patient provided that one skilled in the art can nevertheless determine information about the patient from the sample. It is also understood that extraneous material or information that is not the sample could be utilized to conclusively link the patient to the sample. For a non-limiting example, a double blind test requires a chart or database to match a sample with a patient.

As used herein by the term "patient" it is meant a biologic organism that is the subject of an assay to determine information about the organism. While, in most embodiments of the methods disclosed herein the patient is a human, the methods are not limited for use with an individual human. The patient can be a group of humans. The patient can also be part of a human. For non-limiting examples of this embodiment, the patient could be a tissue sample not linked to a human body, or a transformed cell line. In certain embodiments the patient might also a non-human organism.

As used herein the term "reference value" it is meant a value that statistically correlates to a particular outcome when compared to an assay result. In preferred embodiments the reference value is determined from statistical review of studies that compare HE4a expression with known clinical outcomes. Some such studies are presented in the Examples section herein. However, studies from the literature and the experience of users of the methods disclosed herein can also be used to produce or adjust a reference value. Reference values can also be determined from consideration of cases and results that are particularly relevant to the patient's medical history, genetics, age and other factors.

As used herein the term "body fluid" it is meant a material obtained from a patient that is substantially fluid in consistency, but may have solid or particulate matter associated with it. A body fluid can also contain material and portions that are not from the patient. For instance a body fluid can be diluted with water, or can contain preservative, such as EDTA. Non-limiting examples of body fluids blood, serum, serosal fluids, plasma, lymph, urine, cerebrospinal fluid, saliva, mucosal secretions of the secretory tissues and organs, vaginal secretions, breast milk, tears, and ascites fluids such as those associated with non-solid tumors. Additional examples include fluids of the pleural, pericardial, peritoneal, abdominal and other body cavities, and the like. Biological fluids may further include liquid solutions contacted with a subject or biological source, for example, cell and organ culture medium including cell or organ conditioned medium, lavage fluids and the like.

A sample is obtained from a patient "during treatment" if the sample is obtained as a prelude to administration, at the time of administration, or following administration of a therapeutic composition or method or during the period of follow-up that occurs thereafter. Use of this term explicitly encompasses situations in which samples are obtained prior to and after administration of the treatment (i.e., to assess effectiveness of the treatment or recurrence), as well as situations in which multiple samples are taken intermittently during an extended course of treatment.

Detailed Description

The methods disclosed herein pertain to HE4a, a member of the "four-disulfide core" family of proteins as described herein. The "four-disulfide core" family of proteins comprises a heterogeneous group of small acid- and heat-stable molecules of divergent function and which includes human epididymal four-disulfide core protein, or "HE4" (Kirchhoff et al., 1991 Biol. Reprod. 45:350-357; Wang et al., 1999 Gene 229:101; Schummer et al., 1999 Gene 238:375).

HE4 cDNA was first isolated from human epididymis (Kirchhoff et al., 1991 Biol. Reprod. 45:350-357), and HE4 cDNA was later detected with high frequency in cDNA libraries constructed from ovarian carcinomas (Wang et al., 1999 Gene 229:101; Schummer et al., 1999 Gene 238:375). HE4a, a new member of the "four-disulfide core" family of proteins was described in U.S. patent application publication number 2003/0108965 A1, U.S. patent application no. 10/233,150, which is incorporated herein by reference, including the SEQ ID NO designations used in this disclosure. HE4a exhibits a sequence that is highly similar to, but distinct from, HE4.

For the purposes of this disclosure, detection of either HE4 or HE4a are considered synonymous, and detection of either molecule can be used in the methods described herein. It is not material whether HE4a is a molecule distinct from HE4 or whether the sequence of HE4a merely represents a correction of the published HE4 sequence.

The methods disclosed herein also pertain in part to compositions and methods for detection of cell surface and/or soluble forms of HE4a that occur naturally in subjects, including elevated levels of such polypeptides in subjects having certain carcinomas (e.g., endometrial carcinomas). This disclosure therefore provides useful compositions and methods for the detection and diagnosis of a malignant condition in a subject by specific detection of such cell surface and/or soluble HE4a polypeptides.

According to the methods disclosed herein, a soluble human HE4a antigen polypeptide (or HE4a polypeptide) can be detected in a biological sample from a subject or biological source. Biological samples may be provided by obtaining a blood sample, biopsy specimen, tissue explant, organ culture, biological fluid or any other tissue or cell preparation from a subject or a biological source. The subject or biological source may be a human or non-human animal, a primary cell culture or culture adapted cell line including but not limited to genetically engineered cell lines that may contain chromosomally integrated or episomal recombinant nucleic acid sequences, immortalized or immortalizable cell lines, somatic cell hybrid cell lines, differentiated or differentiable cell lines, transformed cell lines and the like. In certain preferred embodiments of the methods disclosed herein, the subject or biological source may be suspected of having or being at risk for having a malignant condition, which in certain further preferred embodiments may be an endometrial cancer such as an endometrial carcinoma and in certain other preferred embodiments of the methods disclosed herein, the subject or biological source may be known to be free of a risk or presence of such diseases.

In some embodiments, the biological sample includes at least one cell from a subject or biological source, and in certain other preferred embodiments the biological sample is a biological fluid containing another tumor marker, such as CA-125 or a soluble human mesothelin related antigen polypeptide. Biological fluids are typically liquids at physiological temperatures and may include naturally occurring fluids present in, withdrawn from, expressed or otherwise extracted from a subject or biological source. Certain biological fluids derive from particular tissues, organs or localized regions and certain other biological fluids may be more globally or systemically situated in a subject or biological source. Non-limiting examples of biological fluids include blood, serum and serosal fluids, plasma, lymph, urine, cerebrospinal fluid, saliva, mucosal secretions of the secretory tissues and organs, vaginal secretions, breast milk, tears, and ascites fluids such as those associated with non-solid tumors. Additional examples include fluids of the pleural, pericardial, peritoneal, abdominal and other body cavities, and the like. Biological fluids may further include liquid solutions contacted with a subject or biological source, for example, cell and organ culture medium including cell or organ conditioned medium, lavage fluids and the like. In other preferred embodiments the biological sample is a cell-free liquid solution, such as blood serum, plasma, or the supernatant of centrifuged urine.

In certain other preferred embodiments the biological sample comprises an intact cell, and in certain other preferred embodiments the biological sample comprises a cell extract containing a nucleic acid sequence encoding a HE4a antigen polypeptide having the amino acid sequence set forth in SEQ ID NOS: 11 or 13, or a fragment or variant thereof. In still other embodiments of the methods disclosed herein, it is desired that cells are physically or chemically ruptured or lysed before assaying to provide cell contents for analysis.

A "molecule naturally occurring in soluble form" in a sample may be a soluble protein, polypeptide, peptide, amino acid, or derivative thereof; a lipid, fatty acid or the like, or derivative thereof; a carbohydrate, saccharide or the like or derivative thereof, a nucleic acid, nucleotide, nucleoside, purine, pyrimidine or related molecule, or derivative thereof, or the like; or any combination thereof such as, for example, a glycoprotein, a glycolipid, a lipoprotein, a proteolipid, or any other biological molecule that is a soluble or cell-free constituent of a biological sample as provided herein. A "molecule naturally occurring in soluble form" further refers to a molecule that is in solution or present in a biological sample, including a biological fluid as provided herein, and that is not bound to the surface of an intact cell. For example, a molecule naturally occurring in soluble form may include but need not be limited to a solute; a component of a macromolecular complex; a material that is shed, secreted or exported from a cell; a colloid; a microparticle or nanoparticle or other fine suspension particle; or the like.

The presence of a malignant condition in a subject refers to the presence of dysplastic, cancerous and/or transformed cells in the subject, including, for example neoplastic, tumor, non-contact inhibited or oncogenically transformed cells, or the like. By way of illustration and not limitation, in the context of the methods disclosed herein a malignant condition may refer further to the presence in a subject of cancer cells that are capable of secreting, shedding, exporting or releasing a HE4a antigen polypeptide (or a HE4a polypeptide) in such a manner that elevated levels of such a polypeptide are detectable in a biological sample from the subject. In preferred embodiments, for example, such cancer cells are malignant epithelial cells such as carcinoma cells, and in particularly preferred embodiments such cancer cells are malignant mesothelioma cells, which are transformed variants of squamous cell epithelial or mesothelial cells that are found, for example, lining pleural, pericardial, peritoneal, abdominal and other body cavities.

In the most preferred embodiments of the methods disclosed herein, tumor cells, the presence of which signifies the presence of a malignant condition, are endometrial carcinoma cells, including primary and metastatic endometrial carcinoma cells. Criteria for classifying a malignancy as endometrial carcinoma are well known in the art as are the establishment and characterization of human endometrial carcinoma cell lines from primary and metastatic tumors. In other embodiments, the malignant condition may be mesothelioma, pancreatic carcinoma, non-small cell lung carcinoma or another form of cancer, including any of the various carcinomas such as squamous cell carcinomas and adenocarcinomas, and also including sarcomas and hematologic malignancies (e.g., leukemias, lymphomas, myelomas, etc.). Classification of these and other malignant conditions is known to those having familiarity with the art, and the present disclosure provides determination of the presence of a HE4a polypeptide in such a malignant condition without undue experimentation.

Reference values are provided in the examples contained herein. Such values are suitable for practice of the methods disclosed herein. However it should be noted that the use of the methods disclosed herein is not limited to those reference values or that data. Those skilled in the art can obtain a reference value for their particular needs. Such a reference value can be obtained by analyzing HE4a express of patients as they undergo biopsy procedures for uterine or endometrial masses suspected of being malignant. Methods of obtaining such reference valus are contained herein and provided in the examples. Users of the methods disclosed herein may wish to obtain different reference value than provided herein to focus on specific categories of patients. In is foreseen that such categories could include age, genetic background, risk of cancer, medical history, blood type, physical characteristics such as body mass, and other categories.

As provided herein, the method of screening for the presence of a malignant condition in a subject may feature the use of an antibody specific for a HE4a antigen polypeptide or an antibody specific for a HE4a polypeptide.

Antibodies that are specific for a HE4a antigen polypeptide (or a HE4a polypeptide) are readily generated as monoclonal antibodies or as polyclonal antisera, or may be produced as genetically engineered immunoglobulins (Ig) that are designed to have desirable properties using methods well known in the art. For example, by way of illustration and not limitation, antibodies may include recombinant IgGs, chimeric fusion proteins having immunoglobulin derived sequences or "humanized" antibodies (see, e.g., U.S. Pat. Nos. 5,693,762; 5,585,089; 4,816,567; 5,225,539; 5,530,101; and references cited therein) that may all be used for detection of a human HE4a polypeptide according to the methods disclosed herein. Such antibodies may be prepared as provided herein, including by immunization with HE4a polypeptides as described below. For example, as provided herein, nucleic acid sequences encoding HE4a polypeptides are disclosed, such that those skilled in the art may routinely prepare these polypeptides for use as immunogens. For instance, monoclonal antibodies such as 2H5, 3D8 and 4H4, which are described in greater detail below, may be used to practice certain methods according to the methods disclosed herein.

The term "antibodies" includes polyclonal antibodies, monoclonal antibodies, fragments thereof such as F(ab')₂, and Fab fragments, as well as any naturally occurring or recombinantly produced binding partners, which are molecules that specifically bind a HE4a polypeptide. Antibodies are defined to be "immunospecific" or specifically binding if they bind HE4a polypeptide with a Kₐ of greater than or equal to about 10.sup.4 M⁻¹, preferably of greater than or equal to about 10⁵ M⁻¹, more preferably of greater than or equal to about 106 M⁻¹ and still more preferably of greater than or equal to about 10⁷ M⁻¹. Affinities of binding partners or antibodies can be readily determined using conventional techniques, for example those described by Scatchard et al., Ann. N.Y. Acad. Sci. 51:660 (1949). Determination of other proteins as binding partners of a HE4a polypeptide can be performed using any of a number of known methods for identifying and obtaining proteins that specifically interact with other proteins or polypeptides, for example, a yeast two-hybrid screening system such as that described in U.S. Pat. No. 5,283,173 and U.S. Pat. No. 5,468,614, or the equivalent. The methods disclosed herein also includes the use of a HE4a polypeptide, and peptides based on the amino acid sequence of a HE4a polypeptide, to prepare binding partners and antibodies that specifically bind to a HE4a polypeptide.

Antibodies may generally be prepared by any of a variety of techniques known to those of ordinary skill in the art (see, e.g., Harlow and Lane, Antibodies: A Laboratory Manual, Cold Spring Harbor Laboratory, 1988). In one such technique, an immunogen comprising a HE4a polypeptide, for example a cell having a HE4a polypeptide on its surface or an isolated HE4a polypeptide is initially injected into a suitable animal (e.g., mice, rats, rabbits, sheep and goats), preferably according to a predetermined schedule incorporating one or more booster immunizations, and the animals are bled periodically. Polyclonal antibodies specific for the HE4a polypeptide may then be purified from such antisera by, for example, affinity chromatography using the polypeptide coupled to a suitable solid support.

Monoclonal antibodies specific for HE4a polypeptides or variants thereof may be prepared, for example, using the technique of Kohler and Milstein (1976 Eur. J. Immunol. 6:511-519), and improvements thereto. Briefly, these methods involve the preparation of immortal cell lines capable of producing antibodies having the desired specificity (i.e., reactivity with the mesothelin polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. For example, the spleen cells and myeloma cells may be combined with a membrane fusion promoting agent such as polyethylene glycol or a nonionic detergent for a few minutes, and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and tested for binding activity against the polypeptide. Hybridomas having high reactivity and specificity are preferred. Hybridomas that generate monoclonal antibodies that specifically bind to HE4a polypeptides are contemplated by the methods disclosed herein.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse or other suitable host. Monoclonal antibodies may then be harvested from the ascites fluid or the blood. Contaminants may be removed from the antibodies by conventional techniques, such as chromatography, gel filtration, precipitation, and extraction. For example, antibodies may be purified by chromatography on immobilized Protein G or Protein A using standard techniques.

Within certain embodiments, the use of antigen-binding fragments of antibodies may be preferred. Such fragments include Fab fragments, which may be prepared using standard techniques (e.g., by digestion with papain to yield Fab and Fc fragments). The Fab and Fc fragments may be separated by affinity chromatography (e.g., on immobilized protein A columns), using standard techniques. Such techniques are well known in the art, see, e.g., Weir, D. M., Handbook of Experimental Immunology, 1986, Blackwell Scientific, Boston.

Multifunctional fusion proteins having specific binding affinities for preselected antigens by virtue of immunoglobulin V-region domains encoded by DNA sequences linked in-frame to sequences encoding various effector proteins are known in the art, for example, as disclosed in EP-B1-0318554, U.S. Pat. No. 5,132,405, U.S. Pat. No. 5,091,513 and U.S. Pat. No. 5,476,786. Such effector proteins include polypeptide domains that may be used to detect binding of the fusion protein by any of a variety of techniques with which those skilled in the art will be familiar, including but not limited to a biotin mimetic sequence (see, e.g.; Luo et al., 1998 J. Biotechnol. 65:225 and references cited therein), direct covalent modification with a detectable labeling moiety, non-covalent binding to a specific labeled reporter molecule, enzymatic modification of a detectable substrate or immobilization (covalent or non-covalent) on a solid-phase support.

Single chain antibodies for use in the methods disclosed herein may also be generated and selected by a method such as phage display (see, e.g., U.S. Pat. No. 5,223,409; Schlebusch et al., 1997 Hybridomas 16:47; and references cited therein). Briefly, in this method, DNA sequences are inserted into the gene III or gene VIII gene of a filamentous phage, such as M13. Several vectors with multicloning sites have been developed for insertion (McLafferty et al., Gene 128:29-36, 1993; Scott and Smith, Science 249:386-390, 1990; Smith and Scott, Methods Enzymol. 217:228-257, 1993). The inserted DNA sequences may be randomly generated or may be variants of a known binding domain for binding to a HE4a polypeptide. Single chain antibodies may readily be generated using this method. Generally, the inserts encode from 6 to 20 amino acids. The peptide encoded by the inserted sequence is displayed on the surface of the bacteriophage. Bacteriophage expressing a binding domain for a HE4a polypeptide are selected by binding to an immobilized HE4a polypeptide, for example a recombinant polypeptide prepared using methods well known in the art and nucleic acid coding sequences as disclosed herein. Unbound phage are removed by a wash, typically containing 10 mM Tris, 1 mM EDTA, and without salt or with a low salt concentration. Bound phage are eluted with a salt containing buffer, for example. The NaCl concentration is increased in a step-wise fashion until all the phage are eluted. Typically, phage binding with higher affinity will be released by higher salt concentrations. Eluted phage are propagated in the bacteria host. Further rounds of selection may be performed to select for a few phage binding with high affinity. The DNA sequence of the insert in the binding phage is then determined. Once the predicted amino acid sequence of the binding peptide is known, sufficient peptide for use herein as an antibody specific for a HE4a polypeptide may be made either by recombinant means or synthetically. Recombinant means are used when the antibody is produced as a fusion protein. The peptide may also be generated as a tandem array of two or more similar or dissimilar peptides, in order to maximize affinity or binding

To detect an antigenic determinant reactive with an antibody specific for a HE4a polypeptide, the detection reagent is typically an antibody, which may be prepared as described herein. There are a variety of assay formats known to those of ordinary skill in the art for using an antibody to detect a polypeptide in a sample, including but not limited to enzyme linked immunosorbent assay (ELISA), radioimmunoassay (RIA), immunofluorimetry, immunoprecipitation, equilibrium dialysis, immunodiffusion and other techniques. See, e.g., Harlow and Lane, Antibodies: A Laboratory Manual, Cold Spring Harbor Laboratory, 1988; Weir, D. M., Handbook of Experimental Immunology, 1986, Blackwell Scientific, Boston. For example, the assay may be performed in a Western blot format, wherein a protein preparation from the biological sample is submitted to gel electrophoresis, transferred to a suitable membrane and allowed to react with the antibody. The presence of the antibody on the membrane may then be detected using a suitable detection reagent, as is well known in the art and described below.

In another embodiment, the assay involves the use of an antibody immobilized on a solid support to bind to the target HE4a polypeptide and remove it from the remainder of the sample. The bound HE4a polypeptide may then be detected using a second antibody reactive with a distinct HE4a polypeptide antigenic determinant, for example, a reagent that contains a detectable reporter moiety. As a non-limiting example, according to this embodiment the immobilized antibody and the second antibody which recognize distinct antigenic determinants may be any two of the monoclonal antibodies described herein selected from the monoclonal antibodies 2H5, 3D8 and 4H4. Alternatively, a competitive assay may be utilized, in which a HE4a polypeptide is labeled with a detectable reporter moiety and allowed to bind to the immobilized HE4a polypeptide specific antibody after incubation of the immobilized antibody with the sample. The extent to which components of the sample inhibit the binding of the labeled polypeptide to the antibody is indicative of the reactivity of the sample with the immobilized antibody, and as a result, indicative of the level of HE4a in the sample.

The solid support may be any material known to those of ordinary skill in the artto which the antibody may be attached, such as a test-well in a microtiter plate, a nitrocellulose filter or another suitable membrane. Alternatively, the support may be a bead or disc, such as glass, fiberglass, latex or a plastic such as polystyrene or polyvinylchloride. The antibody may be immobilized on the solid support using a variety of techniques known to those in the art, which are amply described in the patent and scientific literature.

In certain preferred embodiments, the assay for detection of HE4a antigen polypeptide in a sample is a two-antibody sandwich assay. This assay may be performed by first contacting a HE4a polypeptide-specific antibody (e.g., a monoclonal antibody such as 2H5, 3D8 or 4H4) that has been immobilized on a solid support, commonly the well of a microtiter plate, with the biological sample, such that a soluble molecule naturally occurring in the sample and having an antigenic determinant that is reactive with the antibody is allowed to bind to the immobilized antibody (e.g., a 30 minute incubation time at room temperature is generally sufficient) to form an antigen-antibody complex or an immune complex. Unbound constituents of the sample are then removed from the immobilized immune complexes. Next, a second antibody specific for a HE4a antigen polypeptide is added, wherein the antigen combining site of the second antibody does not competitively inhibit binding of the antigen combining site of the immobilized first antibody to a HE4a polypeptide (e.g., a monoclonal antibody such as 2H5, 3D8 or 4H4 that is not the same as the monoclonal antibody immobilized on the solid support). The second antibody may be detectably labeled as provided herein, such that it may be directly detected. Alternatively, the second antibody may be indirectly detected through the use of a detectably labeled secondary (or "second stage") anti-antibody, or by using a specific detection reagent as provided herein. The methods disclosed herein are not limited to any particular detection procedure, as those having familiarity with immunoassays will appreciate that there are numerous reagents and configurations for immunologically detecting a particular antigen (e.g., a mesothelin polypeptide) in a two-antibody sandwich immunoassay.

In certain preferred embodiments of the methods disclosed herein using the two-antibody sandwich assay described above, the first, immobilized antibody specific for a HE4a antigen polypeptide is a polyclonal antibody and the second antibody specific for a HE4a antigen polypeptide is a polyclonal antibody. Any combination of non-competitive HE4a antibodies could be used with the methods disclosed herein. Including monoclonal antibodies, polyclonal antibodies and combinations thereof. In certain other embodiments of the methods disclosed herein the first, immobilized antibody specific for a HE4a antigen polypeptide is a monoclonal antibody and the second antibody specific for a HE4a antigen polypeptide is a polyclonal antibody. In certain other embodiments of the methods disclosed herein the first, immobilized antibody specific for a HE4a antigen polypeptide is a polyclonal antibody and the second antibody specific for a HE4a antigen polypeptide is a monoclonal antibody. In certain other highly preferred embodiments of the methods disclosed herein the first, immobilized antibody specific for a HE4a antigen polypeptide is a monoclonal antibody and the second antibody specific for a HE4a antigen polypeptide is a monoclonal antibody. For example, in these embodiments it should be noted that monoclonal antibodies 2H5, 3D8 and 4H4 as provided herein recognize distinct and noncompetitive antigenic determinants (e.g., epitopes) on HE4a polypeptides, such that any pairwise combination of these monoclonal antibodies may be employed. In other preferred embodiments of the methods disclosed herein the first, immobilized antibody specific for a HE4a antigen polypeptide and/or the second antibody specific for a HE4a antigen polypeptide may be any of the kinds of antibodies known in the art and referred to herein, for example by way of illustration and not limitation, Fab fragments, F(ab')₂ fragments, immunoglobulin V-region fusion proteins or single chain antibodies. Those familiar with the art will appreciate that the methods disclosed herein encompass the use of other antibody forms, fragments, derivatives and the like in the methods disclosed and claimed herein.

In certain particularly preferred embodiments, the second antibody may contain a detectable reporter moiety or label such as an enzyme, dye, radionuclide, luminescent group, fluorescent group or biotin, or the like. Any reporter moiety or label could be used with the methods disclosed herein so long as the signal of such is directly related or proportional to the quantity of antibody remaining on the support afterwash. The amount of the second antibody that remains bound to the solid support is then determined using a method appropriate for the specific detectable reporter moiety or label. For radioactive groups, scintillation counting or autoradiographic methods are generally appropriate. Antibody-enzyme conjugates may be prepared using a variety of coupling techniques (for review see, e.g., Scouten, W. H., Methods in Enzymology 135:30-65, 1987). Spectroscopic methods may be used to detect dyes (including, for example, colorimetric products of enzyme reactions), luminescent groups and fluorescent groups. Biotin may be detected using avidin or streptavidin, coupled to a different reporter group (commonly a radioactive or fluorescent group or an enzyme). Enzyme reporter groups may generally be detected by the addition of substrate (generally for a specific period of time), followed by spectroscopic, spectrophotometric or other analysis of the reaction products. Standards and standard additions may be used to determine the level of antigen in a sample, using well known techniques.

In another embodiment, the methods disclosed herein contemplate the use of a HE4a antigen polypeptide as provided herein to screen for the presence of a malignant condition by detection of immunospecifically reactive antibodies in a biological sample from a biological source or subject. According to this embodiment, a HE4a antigen polypeptide (or a fragment or variant thereof including a truncated HE4a antigen polypeptide as provided herein) is detectably labeled and contacted with a biological sample to detect binding to the HE4a antigen polypeptide of an antibody naturally occurring in soluble form in the sample. For example, the HE4a antigen polypeptide may be labeled biosynthetically by using the sequences disclosed herein in concert with well known methods such as incorporation during in vitro translation of a readily detectable (e.g. radioactively labeled) amino acid, or by using other detectable reporter moieties such as those described above. Without wishing to be bound by theory, this embodiment of the methods disclosed herein contemplate that certain HE4a polypeptides such as the HE4a fusion polypeptides disclosed herein, may provide peptides that are particularly immunogenic and so give rise to specific and detectable antibodies. For example, according to this theory certain HE4a fusion polypeptides may represent "non-self' antigens that provoke an avid immune response, while HE4a polypeptides that lack fusion domains may be viewed by the immune system as more resembling "self' antigens that do not readily elicit humoral or cell-mediated immunity.

As noted above, the methods disclosed herein pertain in part to the surprising finding that soluble forms of HE4a antigen polypeptides occur naturally in subjects, including elevated levels of such soluble HE4a polypeptides in subjects having certain carcinomas.

A method of screening for the presence of a malignant condition according to the methods disclosed herein can be further enhanced by the detection of more than one tumor associated marker in a biological sample from a subject. Accordingly, certain embodiments the methods disclosed herein provide a method of screening that, in addition to detecting reactivity of a naturally occurring soluble sample component with an antibody specific for a HE4a antigen polypeptide, also includes detection of at least one additional soluble marker of a malignant condition using established methods as known in the art and provided herein. As noted above, there are currently a number of soluble tumor associated antigens that are detectable in samples of readily obtained biological fluids. For example, certain embodiments of the methods disclosed herein relate to human mesothelin polypeptides, which include polypeptides such as the novel soluble mesothelin related antigen (MRA) polypeptide described in Scholler et al. (1999 Proc. Nat. Acad. Sci. USA 96:11531) and as also described in U.S. Patent 6,770,445.

As provided herein, a "mesothelin polypeptide" is a soluble polypeptide having an amino acid sequence that includes the peptide:

1 EVEKTACPSGKKAREIDES SEQ ID NO:14

and further having at least one antigenic determinant reactive with at least one antibody having an antigen combining site that competitively inhibits the immunospecific binding of MAb K-1 (Chang et al., 1996 Proc. Nat. Acad. Sci. USA 93:136; MAb K-1 is available from, e.g., Signet Laboratories, Inc., Dedham, Mass.) or of monoclonal antibodies OV569, 4H3, 3G3 or 1A6 as provided in U.S. Patent 6,770,445.

Thus, these additional soluble tumor associated antigens for use according to the methods disclosed herein can include, but need not be limited to, mesothelin and mesothelin related antigen, CEA, CA125, sialyl TN, SCC, TPS and PLAP, (see e.g., Bast et al., 1983 N. Eng. J. Med. 309:883; Lloyd et al., 1997 Int. J. Canc. 71:842; Sarandakou et al., 1997 Acta Oncol. 36:755; Sarandakou et al., 1998 Eur. J. Gynaecol. Oncol. 19:73; Meier et al., 1997 Anticanc. Res. 17(4B):2945; Kudoh et al., 1999 Gynecol. Obstet. Invest. 47:52; Ind et al., 1997 Br. J. Obstet. Gynaecol. 104:1024; Bell et al. 1998 Br. J. Obstet. Gynaecol. 105:1136; Cioffi et al., 1997 Tumori 83:594; Meier et al. 1997 Anticanc. Res. 17(4B):2949; Meier et al., 1997 Anticanc. Res. 17(4B):3019) and may further include any known marker the presence of which in a biological sample may be correlated with the presence of at least one malignant condition, as provided herein.

Alternatively, nucleic acid sequences encoding HE4a polypeptides may be detected, using standard hybridization and/or polymerase chain reaction (PCR) techniques. Suitable probes and primers may be designed by those of ordinary skill in the art based on the HE4a cDNA sequences provided herein. Assays may generally be performed using any of a variety of samples obtained from a biological source, such as eukaryotic cells, bacteria, viruses, extracts prepared from such organisms and fluids found within living organisms.

EXAMPLES

General methods

Standard recombinant DNA and molecular cloning techniques used in the Examples are well known in the art and are described by Sambrook, J., Fritsch, E. F. and Maniatis, T. Molecular Cloning: A Laboratory Manual; Cold Spring Harbor Laboratory Press: Cold Spring Harbor, (1989) (Maniatis) and by T. J. Silhavy, M. L. Bennan, and L. W. Enquist, Experiments with Gene Fusions, Cold Spring Harbor Laboratory, Cold Spring Harbor, N.Y. (1984) and by Ausubel, F. M. et al., Current Protocols in Molecular Biology, pub. by Greene Publishing Assoc. and Wiley-Interscience (1987).

The meaning of abbreviations is as follows: "h" means hour(s), "min" means minute(s), "sec" means second(s), "d" means day(s), "mL" means milliliters, "L" means liters, "U" means units, "pM" means picomolar, "ROC" means receiver operating characteristic, "AUC" means area under curve, "StDev" means standard deviation, "Min" means minimum "Max" means maximum and "p" means p-value.

The subject matter of this disclosure is now described with reference to the following Examples. These Examples are provided for the purpose of illustration only, and the subject matter is not limited to these Examples, but rather encompasses all variations which are evident as a result of the teaching provided herein.

Example 1

Utility of a novel serum tumor marker HE4 versus CA-125 in patients with endometrial cancer.

There are approximately 40,880 new cases of uterine cancer diagnosed in the US annually resulting in 7,310 deaths. Endometrial cancer is surgically staged with approximately 75% of patients presenting with stage I disease. Serum levels of CA-125 are elevated in 75% of patients presenting with advanced stage disease and in only 17% of patients with early stage disease. The use of CA-125 for the detection of recurrent disease is limited at best. Only 25% of patients with asymptomatic recurrent disease have an elevated CA-125. A better marker indicating early recurrent disease is needed. The objective of this study was to examine the value of a novel serum tumor marker HE4 and to compare it to CA-125 as a marker for endometrial cancer.

The methods used in this example are now described.

This was a prospective IRB approved study comparing HE4 and CA-125 in patients that were to undergo surgery with removal of their uterus and ovaries for either benign disease or uterine cancer. Informed consent was obtained from all patients. Blood samples were drawn pre-operatively and serum was collected and frozen down to -80 C within four hours of being drawn. HE4 and CA-125 levels were determined using assays from Fujirebio Diagnostics Inc. Surgical pathologic results were then compared to the tumor marker levels. ROC curves were constructed for each tumor maker.

The results of the experiments of this example are now described.

A total of 221 patients were enrolled on protocol; 128 benign cases 93 endometrial cancers. Endometrial cancer patients had the following surgical stages; 62 stage I, 4 stage II, 19 stage III and 8 stage IV. The mean HE4 for endometrial cancer patients was 161pM (15 to 1052) and for patients with benign disease 49.3pM (14 to 254). The mean CA-125 for patients with endometrial cancer was 52U/ml (3 to 1427) and benign disease was 46U/ml (3-773). The ROC curve with AUC were calculated for HE4 and CA-125 and compared for each category (Table 1).

**Table 1**

| ROC Curve AUC | | | |
|---|---|---|---|
| | HE4a (AUC) | CA-125 (AUC) | p-value |
| Benign vs. Endometrial | 79% | 54% | <0.001 |
| Benign vs. Stage I | 76% | 48% | <0.001 |
| Benign vs. Stage II | 88% | 69% | 0.253 |
| Benign vs. Stage III | 86% | 66% | 0.002 |
| Benign vs. Stage IV | 86% | 66% | 0.006 |

From the information provided herein, it can be concluded that HE4 is elevated in all stages of endometrial cancer and has a greater sensitivity than CA-125 for the detection of endometrial cancer in all stages. HE4 can be used as a marker for early recurrent disease in patients with endometrial cancer.

Example 2

The methods used in this example are now described.

Serum sample were obtained from patients enrolled into a prospective IRB approved protocol at Women and Infants Hospital / Brown University and from the IRB approved serum bank at The University of Texas MD Anderson Cancer Center. All patients entered onto protocol signed informed consent prior to collection of the study blood samples. All blood samples were collected in the immediate preoperative period centrifuged and serum drawn off and frozen to -80°C with in 4 hours of collection. All patients subsequently underwent surgery consisting of an exploratory laparotomy with hysterectomy bilateral salpingooophorectomy with full surgical staging including washing, pelvic and periaortic lymphadenectomies. Serum samples obtained from Women and Infants' Hospital were analyzed at the laboratories of Fujirebio Diagnostics Inc. The samples obtained from MD Anderson Cancer Center were analyzed on site. Normal control samples were obtained from healthy patients. Serum tumor marker levels for CA125, HE4, CA72,4 SMRP and urinary SMRP levels were determined using assays from Fujirebio Diagnostic Inc. ROC curves were constructed for each tumor marker and the sensitivity at a set specificity of 95% was determined.

The results of this example are now described

Two hundred and thirty three serum sample from patients with surgically stage endometrial cancer were obtained along with 156 serum samples from healthy controls.

In the group of patients with surgically staged endometrial cancer there were 151 stage I, 21 stage II, 47 stage III and 14 stage IV. The mean serum tumor marker levels for each marker analyzed were calculated for the healthy controls, surgical stage I patients and surgical stage II-IV patients and are shown in (Table 2). The area under the ROC curves (ROC-AUC) for CA125, HE4, CA72.4 SMRP and urinary SMRP levels were determined for each marker (Table 3). Logistic regression analysis was used to compare each tumor marker independently and in various combinations at set specificities of 90%, 95% and 98% for endometrial cancer sample versus controls(Tables 4,5 and 6). The sensitivity for differentiation of controls versus endometrial cancer for individual markers revealed HE4 to perform the best. For all stages of endometrial cancer HE4 had a sensitivity of 44.9% at a set specificity of 95% compared to 25.2% for CA125 (p=0.0001). For stage I cases, HE4 showed an improvement in the sensitivity of 20.5% compared to CA125 alone (37.1% vs 16.6%, p=0.0001) (Table 3). For patients with stage II-IV disease the sensitivity for HE4 increased to 59.8% with a set specificity of 95% an increase of 18.3% over that of CA125 (p=0.0001)

When analyzing combination of any two markers the coupling of HE4 and CA125 or HE4 and urinary SMRP increase the sensitivity to 48.7 and 49.6 respectively for all stages of endometrial cancer. Analysis of the combination of HE4 and CA125 in patients with stage II-IV disease resulted in a sensitivity of 69.5% at a set specificity of 95%. This represented a 24.4% increase in sensitivity over CA125 alone (p=0.0001). The combination of HE4, CA125 and Urinary SMRP yielded the largest gain in sensitivity of 26.1% over that of CA125 alone in all endometrial cancer patients. However, this was only a 2.6% gain in sensitivity over the combination of HE4 and CA125 and was not significantly significant. HE4 was also found to have a higher sensitivity in patients with stage I endometrial cancer over that of CA125 with a sensitivity of 37.1% versus 16.6% respectively. Again the combination of HE4 and CA-125 increased the sensitivity to 39.1% over CA125. However, the combination of HE4, CA125 and Urinary SMRP preformed the best in the patients with stage I disease with a sensitivity of 44.7% increasing the sensitivity over that of CA125 alone by 28.1% (p=0.0001).

**Table 2**

| **Diagnosis** | **Serum CA125** | | | | | |
|---|---|---|---|---|---|---|
| | **N** | **Average** | **StDev** | **Min** | **Max** | **Median** |
| **Normal** | 156 | 13.9 | 9.8 | 3.3 | 73.6 | 11.2 |
| **Endometrial Ca (Stage I)** | 151 | 22.4 | 35.4 | 3.0 | 402.2 | 13.9 |
| **Endometrial Ca (Stage II-IV)** | 82 | 75.5 | 178.5 | 1.8 | 1426.6 | 24.0 |
| **All Endometrial Cancer**s | 234 | 41.0 | 112.0 | 1.8 | 1426.6 | 16.5 |

| **Diagnosis** | **Serum HE4** | | | | | |
|---|---|---|---|---|---|---|
| | **N** | **Average** | **StDev** | **Min** | **Max** | **Median** |
| **Normal** | 156 | 39.7 | 18.7 | 18.0 | 127.8 | 35.4 |
| **Endometrial Ca (Stage I)** | 151 | 107.9 | 172.3 | 1.1 | 1523.0 | 61.0 |
| **Endometrial Ca (Stage II-IV)** | 82 | 221.6 | 487.9 | 9.2 | 4062.7 | 95.4 |
| **All Endometrial Cancers** | 234 | 147.3 | 323.9 | 1.1 | 4062.7 | 70.7 |

| **Diagnosis** | **Serum CA72-4** | | | | | |
|---|---|---|---|---|---|---|
| | **N** | **Average** | **StDev** | **Min** | **Max** | **Median** |
| **Normal** | 153 | 3.0 | 3.6 | 0.9 | 24.4 | 1.6 |
| **Endometrial Ca (Stage I)** | 151 | 4.4 | 22.8 | 0.8 | 280.3 | 1.8 |
| **Endometrial Ca (Stage II-IV)** | 82 | 5.3 | 11.1 | 0.5 | 69.5 | 2.4 |
| **All Endometrial Cancers** | 234 | 4.7 | 19.5 | 0.5 | 280.3 | 1.9 |

| **Diagnosis** | **Serum SMRP** | | | | | |
|---|---|---|---|---|---|---|
| | **N** | **Average** | **StDev** | **Min** | **Max** | **Median** |
| **Normal** | 156 | 0.7 | 0.4 | 0.1 | 2.2 | 0.6 |
| **Endometrial Ca (Stage I)** | 151 | 0.8 | 1.2 | 0.0 | 13.3 | 0.6 |
| **Endometrial Ca (Stage II-IV)** | 82 | 0.6 | 0.5 | 0.0 | 3.4 | 0.6 |
| **All Endometrial Cancers** | 234 | 0.8 | 1.0 | 0.0 | 13.3 | 0.6 |

| **Diagnosis** | **Urine SMRP** | | | | | |
|---|---|---|---|---|---|---|
| | **N** | **Average** | **StDev** | **Min** | **Max** | **Median** |
| **Normal** | 104 | 0.0 | 0.1 | 0.0 | 0.5 | 0.0 |
| **Endometrial Ca (Stage I)** | 150 | 0.3 | 0.6 | 0.0 | 4.0 | 0.1 |
| **Endometrial Ca (Stage II-IV)** | 81 | 0.4 | 0.9 | 0.0 | 4.7 | 0.1 |
| **All Endometrial Cancers** | 232 | 0.3 | 0.7 | 0.0 | 4.7 | 0.1 |

**Table 3**

| **Marker** | **ROC-AUC (Normal vs. Endometrial Ca)** | | |
|---|---|---|---|
| | **Stage I** | **Stage II-IV** | **I All Stages** |
| **CA125** | 61.5% | 76.3% | 66.7% |
| **HE4** | 76.5% | 83.5% | 78.7% |
| **CA72-4** | 49.5% | 60.9% | 53.5% |
| **SMRP** | 52.2% | 50.6% | 51.0% |
| **Urine SMRP** | 70.4% | 74.2% | 71.6% |
| **CA125 & HE4** | 76.4% | 85.9% | 79.1% |
| **CA125 & CA72-4** | 60.8% | 76.7% | 66.2% |
| **CA125 & SMRP** | 61.0% | 79.3% | 66.9% |
| **CA125 & Urine SMRP** | 75.9% | 81.5% | 77.6% |
| **HE4 & CA72-4** | 76.8% | 83.6% | 78.9% |
| **HE4 & SMRP** | 76.6% | 84.4% | 78.8% |
| **HE4 & Urine SMRP** | 77.3% | 84.6% | 79.5% |
| **CA125 & HE4 & CA72-4** | 76.6% | 85.7% | 79.3% |
| **CA125 & HE4 & SMRP** | 76.6% | 88.3% | 79.6% |
| **CA125 & HE4 & Urine SMRP** | 78.0% | 85.9% | 80.3% |
| **CA125 & CA72-4 & SMRP** | 60.1% | 79.6% | 66.2% |
| **HE4 & CA72-4 & SMRP** | 76.8% | 84.3% | 79.0% |
| **HE4 & CA72-4 & Urine SMRP** | 77.4% | 84.7% | 79.5% |
| **CA125 & CA72-4 & Urine SMRP** | 75.3% | 81.2% | 77.1% |
| **CA125 & HE4 & CA72-4 & SMRP** | 76.8% | 88.0% | 79.7% |
| **CA125 & HE4 & CA72-4 & Urine SMRP** | 77.9% | 85.9% | 80.1% |
| **CA125 & HE4 & CA72-4 & SMRP & Urine SMRP** | 78.4% | 89.7% | 81.0% |

**Table 4**

| | **Normal vs. All Endometrial Ca: Sensitivity at** | | |
|---|---|---|---|
| **Marker Combination** | **90% Specificity** | **95% Specificity** | **98% Specificity** |
| **CA125** | 35.9% | 25.2% | 16.7% |
| **HE4** | 55.6% | 44.9% | 35.5% |
| **CA72-4** | 9.4% | 6.8% | 3.4% |
| **SMRP** | 9.8% | 6.8% | 3.4% |
| **Urine SMRP** | 37.5% | 25.9% | 19.8% |
| **CA125 & HE4** | 59.8% | 48.7% | 39.7% |
| **CA125 & CA72-4** | 35.0% | 24.8% | 17.1% |
| **CA125 & SMRP** | 35.0% | 24.4% | 17.1% |
| **CA125 & Urine SMRP** | 44.0% | 35.3% | 24.6% |
| **HE4 & CA72-4** | 56.0% | 44.4% | 33.8% |
| **HE4 & SMRP** | 53.4% | 43.6% | 35.0% |
| **HE4 & Urine SMRP** | 59.9% | 49.6% | 48.3% |
| **CA125 & HE4 & Urine SMRP** | 61.6% | 51.3% | 46.1% |
| **HE4 & CA72-4 & Urine SMRP** | 58.6% | 50.0% | 47.4% |

**Table 5**

| | **Normal vs. All Endometrial Ca (I): Sensitivity at** | | |
|---|---|---|---|
| **Marker Combination** | **98% Specificity** | **95% Specificity** | **98% Specificity** |
| **CA125** | 126.5% | 16.6% | 8.6% |
| **HE4** | 49.0% | 37.1% | 28.5% |
| **CA72-4** | 6.6% | 4.6% | 2.0% |
| **SMRP** | 11.9% | 7.9% | 4.6% |
| **Urine SMRP** | 36.0% | 23.3% | 16.7% |
| **CA125 & HE4** | 51.7% | 39.1% | 29.8% |
| **CA125 & CA72-4** | 26.5% | 16.6% | 10.6% |
| **CA125 & SMRP** | 27.2% | 13.2% | 9.9% |
| **CA125 & Urine SMRP** | 36.0% | 28.7% | 20.0% |
| **HE4 & CA72-4** | 49.7% | 37.1% | 27.2% |
| **HE4 & SMRP** | 47.7% | 37.1% | 27.2% |
| **HE4 & Urine SMRP** | 54.0% | 43.3% | 40.0% |
| **CA125 & HE4 & Urine SMRP** | 56.7% | 44.7% | 40.7% |
| **HE4 & CA72-4 & Urine SMRP** | 54.0% | 42.0% | 40.0% |

**Table 6**

| | **Normal vs. All Endometrial Ca (II-IV): Sensitivity at** | | |
|---|---|---|---|
| **Marker Combination** | **90% Specificity** | **95% Specificity** | **98% Specificity** |
| **CA125** | 53.7% | 41.5% | 31.7% |
| **HE4** | 68.3% | 59.8% | 48.8% |
| **CA72-4** | 14.6% | 11.0% | 6.1% |
| **SMRP** | 6.1% | 4.9% | 1.2% |
| **Urine SMRP** | 40.7% | 30.9% | 25.9% |
| **CA125 & HE4** | 73.2% | 65.9% | 52.4% |
| **CA125 & CA72-4** | 46.3% | 39.0% | 32.9% |
| **CA125 & SMRP** | 56.1% | 40.2% | 29.3% |
| **CA125 & Urine SMRP** | 53.1% | 45.7% | 42.0% |
| **HE4 & CA72-4** | 69.5% | 59.8% | 48.8% |
| **HE4 & SMRP** | 68.3% | 56.1% | 46.3% |
| **HE4 & Urine SMRP** | 69.1% | 67.9% | 59.3% |
| **CA125 & HE4 & Urine SMRP** | 74.1% | 66.7% | 56.8% |
| **HE4 & CA72-4 & Urine SMRP** | 69.1% | 67.9% | 60.5% |

The disclosure of every patent, patent application, and publication cited herein is hereby incorporated herein by reference in its entirety.

While this subject matter has been disclosed with reference to specific embodiments, it is apparent that other embodiments and variations can be devised by others skilled in the art without departing from the true spirit and scope of the subject matter described herein. The appended claims include all such embodiments and equivalent variations.
The present invention will now be described by way of the following clauses:
1. A method of assessing whether a patient is afflicted with an endometrial or uterine cancer, the method comprising assessing expression of HE4 in a sample obtained from the patient, wherein elevated expression of HE4 is an indication that the patient is afflicted with an endometrial or uterine cancer.
2. The method of clause 1, wherein the sample is a solid tissue sample.
3. The method of clause 1, wherein the sample is a body fluid obtained from the patient.
4. The method of clause 3, wherein the body fluid is selected from the group consisting of blood, serum, peritoneal fluid, and vaginal fluid.
5. The method of clause 1, wherein the sample is a fluid that has been contacted with the patient.
6. The method of clause 5, wherein the fluid is selected from the group consisting of a peritoneal wash fluid and a vaginal douche fluid.
7. The method of clause 1, wherein expression of HE4 in the sample is compared with a reference value.
8. The method of clause 1, wherein the reference value corresponds to HE4 expression in patients who are not afflicted with an endometrial or uterine cancer.
9. The method of clause 1, wherein expression of HE4 in the sample is compared with expression of HE4 in an earlier sample obtained at an earlier time from the patient.
10. The method of clause 1, further comprising assessing expression of a second marker selected from the group consisting of CA125 and SMRP in the sample, wherein elevated
   expression of the second marker is a further indication that the patient is afflicted with an endometrial or uterine cancer.
11. The method of clause 1, further comprising assessing expression of both CA125 and SMRP in the sample, wherein elevated expression of either of CA125 and SMRP is a further indication that the patient is afflicted with an endometrial or uterine cancer.
12. A method of assessing the response of a patient afflicted with an endometrial or uterine cancer to a treatment, the method comprising assessing expression of HE4 in samples obtained from the patient at different times during treatment, wherein decreased expression of HE4 at the later time indicates that the patient is responding to the treatment.
13. The method of clause 12, further comprising assessing expression of a second marker selected from the group consisting of CA125 and SMRP in the sample, wherein decreased expression of the second marker is a further indication that the patient is responding to the treatment.
14. The method of clause 12, further comprising assessing expression of both CA125 and SMRP in the sample, wherein decreased expression of either of CA125 and SMRP is a further indication that the patient is responding to the treatment.
15. A method of assessing recurrence in a patient who has been treated for an endometrial or uterine cancer, the method comprising assessing expression of HE4 in samples obtained from the patient following treatment, wherein elevated expression of HE4 indicates that the endometrial or uterine cancer is recurring in the patient.
16. The method of clause 15, further comprising assessing expression of a second marker selected from the group consisting of CA125 and SMRP in the sample, wherein elevated expression of the second marker is a further indication that the endometrial or uterine cancer is recurring in the patient.
17. The method of clause 15, further comprising assessing expression of both CA125 and SMRP in the sample, wherein elevated expression of either of CA125 and SMRP is a further indication that the endometrial or uterine cancer is recurring in the patient.
18. The method of clause 15, wherein expression of HE4 is assessed multiple times following the treatment, and wherein increasing expression of HE4 indicates that the endometrial or uterine cancer is recurring in the patient.
19. A method of assessing the likelihood that a patient will develop an endometrial or uterine cancer, the method comprising assessing expression of HE4 in a sample obtained from the patient, wherein elevated expression of HE4 is correlated with increased likelihood that the patient will develop an endometrial or uterine cancer.
20. The method of clause 19, further comprising assessing expression of a second marker selected from the group consisting of CA125 and SMRP in the sample, wherein elevated expression of the second marker is a further indication that the patient will develop an endometrial or uterine cancer.
21. The method of clause 19, further comprising assessing expression ofboth CA125 and SMRP in the sample, wherein elevated expression of either of CA125 and SMRP is a further indication that the patient will develop an endometrial or uterine cancer.
22. A method of staging a tumor in a patient afflicted with an endometrial or uterine cancer, the method comprising assessing expression of HE4 in a sample obtained from the patient, wherein increasing expression of HE4 indicates a more advanced stage of the cancer.
23. The method of clause 22, further comprising assessing expression of a second marker selected from the group consisting of CA125 and SMRP in the sample, wherein elevated expression of the second marker further indicates a more advanced stage of the cancer.
24. The method of clause 22, further comprising assessing expression of both CA125 and SMRP in the sample, wherein elevated expression of either of CA125 and SMRP further indicates a more advanced stage of the cancer.
25. A method of grading a tumor in a patient afflicted with an endometrial or uterine cancer, the method comprising assessing expression of HE4 in a sample obtained from the patient, wherein increasing expression of HE4 indicates a higher grade of the of the cancer.
26. The method of clause 25, further comprising assessing expression of a second marker selected from the group consisting of CA125 and SMRP in the sample, wherein elevated expression of the second marker further indicates a higher grade of the of the cancer.
27. The method of clause 25, further comprising assessing expression of both CA125 and SMRP in the sample, wherein elevated expression of either of CA125 and SMRP further indicates a higher grade of the of the cancer.
28. A method for assessing the need of a patient diagnosed with an endometrial or uterine cancer for a therapeutic intervention, the method comprising assessing expression of HE4 in a sample obtained from the patient, wherein elevated expression of HE4 indicates need of the patient for the therapeutic intervention.
29. The method of clause 28, further comprising assessing expression of a second marker selected from the group consisting of CA125 and SMRP in the sample, wherein elevated expression of the second marker further indicates need of the patient for the therapeutic intervention.
30. The method of clause 28, wherein the therapeutic intervention is a chemotherapy.

## Claims

1. A method for assessing whether a patient is afflicted with an endometrial or uterine cancer, the method comprising assessing expression of HE4a in a sample obtained from the patient, wherein elevated expression of HE4a is an indication that the patient is afflicted with an endometrial or uterine cancer.

2. The method of claim 1, wherein the sample is a solid tissue sample.

3. The method of claim 1, wherein the sample is a body fluid obtained from the patient, wherein optionally and preferably, the body fluid is selected from the group consisting of blood, serum, peritoneal fluid, and vaginal fluid.

4. The method of claim 1, wherein the sample is a fluid that has been contacted with the patient, wherein optionally and preferably, the fluid is selected from the group consisting of a 20 peritoneal wash fluid and a vaginal douche fluid.

5. The method of claim 1, wherein expression of HE4a in the sample is compared with a reference value.

6. The method of claim 1, wherein the reference value corresponds to HE4a expression in patients who are not afflicted with an endometrial or uterine cancer.

7. The method of claim 1, wherein expression of HE4a in the sample is compared with expression of HE4a in an earlier sample obtained at an earlier time from the patient.

8. The method of claim 1, further comprising:
a) assessing expression of a second marker selected from the group consisting of CA125 and SMRP in the sample, wherein elevated expression of the second marker is a further indication that the patient is afflicted with an endometrial or uterine cancer; or
b) assessing expression of both CA125 and SMRP in the sample, wherein elevated expression of either of CA125 and SMRP is a further indication that the patient is afflicted with an endometrial or uterine cancer.

9. A method for assessing the response of a patient afflicted with an endometrial or uterine cancer to a treatment, the method comprising assessing expression of HE4a in samples obtained from the patient at different times during treatment, wherein decreased expression of HE4a at the later time indicates that the patient is responding to the treatment, wherein optionally and preferably, the method further comprising the step of:
a) assessing expression of a second marker selected from the group consisting of CA125 and SMRP in the sample, wherein decreased expression of the second marker is a further indication that the patient is responding to the treatment; or
b) assessing expression of both CA125 and SMRP in the sample, wherein decreased expression of either of CA125 and SMRP is a further indication that the patient is responding to the treatment.

10. A method for assessing recurrence in a patient who has been treated for an endometrial or uterine cancer, the method comprising assessing expression of HE4a in samples obtained from the patient following treatment, wherein elevated expression of HE4a indicates that the endometrial or uterine cancer is recurring in the patient, wherein optionally and preferably, the method further comprises the step of:
a) assessing expression of a second marker selected from the group consisting of CA125 and SMRP in the sample, wherein elevated expression of the second marker is a further indication that the endometrial or uterine cancer is recurring in the patient; or
b) assessing expression of both CA125 and SMRP in the sample, wherein elevated expression of either of CA125 and SMRP is a further indication that the endometrial or uterine cancer is recurring in the patient.

11. The method of claim 10, wherein expression of HE4a is assessed multiple times following the treatment, and wherein increasing expression of HE4a indicates that the endometrial or uterine cancer is recurring in the patient.

12. A method for assessing the likelihood that a patient will develop an endometrial or uterine cancer, the method comprising assessing expression of HE4a in a sample obtained from the patient, wherein elevated expression of HE4a is correlated with increased likelihood that the patient will develop an endometrial or uterine cancer, wherein optionally and preferably, the method further comprises the step of:
a) assessing expression of a second marker selected from the group consisting of CA125 and SMRP in the sample, wherein elevated expression of the second marker is a further indication that, the patient will develop an endometrial or uterine cancer; or
b) assessing expression of both CA125 and SMRP in the sample, wherein elevated expression of either of CA125 and SMRP is a further indication that the patient will develop an endometrial or uterine cancer.

13. A method for staging a tumor in a patient afflicted with an endometrial or uterine cancer, the method comprising assessing expression of HE4a in a sample obtained from the patient, wherein increasing expression of HE4a indicates a more advanced stage of the cancer, wherein optionally and preferably the method further comprises assessing expression of a second marker selected from the group consisting of CA125 and SMRP in the sample, wherein elevated expression of the second marker further indicates a more advanced stage of the cancer or assessing expression of both CA125 and SMRP in the sample, wherein elevated expression of either of CA125 and SMRP further indicates a more advanced stage of the cancer.

14. A method for grading a tumor in a patient afflicted with an endometrial or uterine cancer, the method comprising assessing expression of HE4a in a sample obtained from the patient, wherein increasing expression of HE4a indicates a higher grade of the of the cancer, wherein optionally and preferably, the method further comprises the step of:
a) assessing expression of a second marker selected from the group consisting of CA125 and SMRP in the sample, wherein elevated expression of the second marker further indicates a higher grade of the of the cancer; or
b) assessing expression of both CA125 and SMRP in the sample, wherein elevated expression of either of CA125 and SMRP further indicates a higher grade of the of the cancer.

15. A method for assessing the need of a patient diagnosed with an endometrial or uterine cancer for a therapeutic intervention, preferably, chemotherapy, the method comprising assessing expression of HE4a in a sample obtained from the patient, wherein elevated expression of HE4a indicates need of the patient for the therapeutic intervention, wherein optionally and preferably, the method further comprises the step of assessing expression of a second marker selected from the group consisting of CA125 and SMRP in the sample, wherein elevated expression of the second marker further indicates need of the patient for the therapeutic intervention.


SEQUENCE LISTING

<110>  FUJIREBIO AMERICA, INC.
       MOORE, Richard
       SOMERS, Elizabeth
       ALLARD, Jeffrey

<120>  USE OF HE4 AND OTHER BIOCHEMICAL MARKERS FOR ASSESSMENT OF
       ENDOMETRIAL AND UTERINE CANCERS

<130>  D4496-00110

<140>  PCT/US07/00215
<141>  2007-01-04

<150>  US 60/756,131
<151>  2006-01-04

<160>  14

<170>  PatentIn version 3.3

<210>  1

<400>  1
000

<210>  2

<400>  2
000

<210>  3

<400>  3
000

<210>  4

<400>  4
000

<210>  5

<400>  5
000

<210>  6

<400>  6
000

<210>  7

<400>  7
000

<210>  8

<400>  8
000

<210>  9

<400>  9
000

<210>  10

<400>  10
000

<210>  11

<400>  11
000

<210>  12

<400>  12
000

<210>  13

<400>  13
000

<210>  14
<211>  19
<212>  PRT
<213>  Artificial

<220>
<223>  Mesothelin Peptide

<400>  14

Glu Val Glu Lys Thr Ala Cys Pro Ser Gly Lys Lys Ala Arg Glu Ile
1               5                   10                  15

Asp Glu Ser